(19) Europäisches Patentamt
European Patent Office
Office européen des brevets

(11) Publication number: 0 292 032 A1

# (12) EUROPEAN PATENT APPLICATION

(21) Application number: 88200717.2

(22) Date of filing: 13.04.88

(51) Int. Cl.4: C07D 213/79 , C07D 213/80 , C07D 213/81 , C07D 215/48 , C07D 221/04 , C07D 471/04 , C07D 491/04 , C07D 211/98 , //(C07D471/04,221:00,209:00), (C07D491/04,307:00,209:00)

The title of the invention has been amended (Guidelines for Examination in the EPO, A-III, 7.3).

(30) Priority: 16.04.87 GB 8709228

(43) Date of publication of application: 23.11.88 Bulletin 88/47

(84) Designated Contracting States: AT BE CH DE ES FR GB GR IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Astles, David Phillip
37 Longridge
Sittingbourne Kent, ME 10 4LN(GB)
Inventor: Flood, Andrew
25 Hollybank Hill London Road
Sittingbourne Kent, ME10 1NF(GB)

(74) Representative: Hunter, Keith Roger Ian et al
4 York Road
London SE1 7NA(GB)

# (54) Process for the preparation of pyridine-2,3-dicarboxylic acid derivatives.

(57) A pyridine compound of formula I

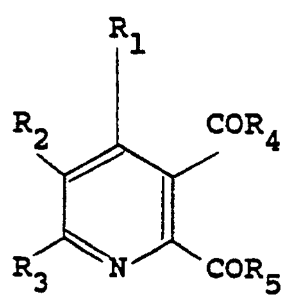

(I)

is prepared from a tetrahydropyridine compound of formula II

EP 0 292 032 A1

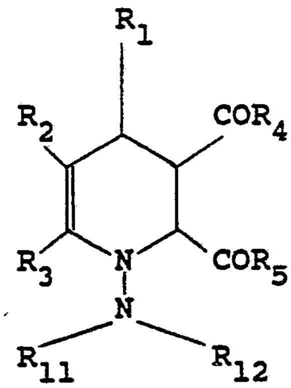

(II)

in a single step or procedure under oxidising conditions. The R meanings are fully defined in the specification but preferably $R_1$ and $R_3$ represent hydrogen, $R_2$, $R_{11}$ and $R_{12}$ represent alkyl, and $R_4$ and $R_5$ represent hydroxy or alkoxy or together represent -O- or $-NR_{10}-$ where $R_{10}$ represents alkyl, cyanoalkyl, aminocarbonylalkyl or phenyl.

## PREPARATION OF HETEROCYCLIC COMPOUNDS

This invention relates to the preparation of certain heterocyclic compounds, namely substituted pyridine compounds.

EP-A-172140 discloses a method for the preparation of an pyridine -2,3-dicarboxylic acid derivative of the formula I

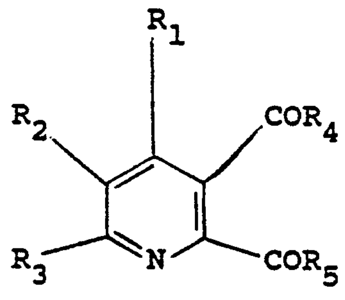

(I)

characterised in that a 1-amino-1,2,3,4-tetrahydropyridine-2,3-dicarboxylic acid derivative of the formula II (the meanings of the substituents $R_1$ to $R_{11}$ being as defined therein)

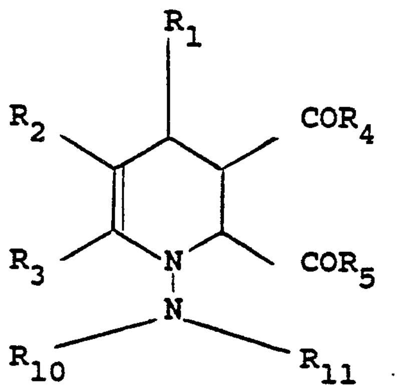

(II)

is subjected to treatment with an acid and/or thermal treatment at at least 20°C to remove $R_{10}R_{11}$ NH to form a 1,4-dihydropyridine-2,3-dicarboxylic acid derivative of the formula III,

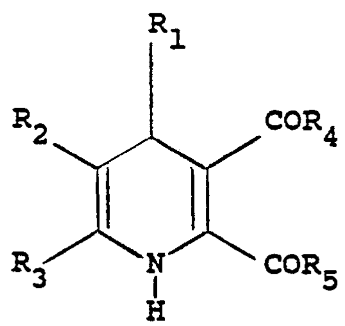

(III)

the 1,4-dihydropyridine-2,3-dicarboxylic acid derivative then being converted into a pyridine-2,3-dicarboxylic acid derivative by oxidation.

Substantially different reaction conditions are described for each step. The first step is said to suitably take place at a temperature in the range 20-200°C, preferably 40-150°C, in an inert solvent, suitably toluene or ethanol. Suitable acids are said to be various organic acids, such as formic, acetic, propionic and oxalic acid, and acid silicates, such as silica gel. Particularly preferred conditions involve heating the

tetrahydropyridine under reflux with about a double quantity of silica gel, in toluene.

In relation to the second step, suitable oxidation agents are said to be, for example, oxygen, hydrogen peroxide, manganese dioxide, chromic acid, solutions of iodine in an organic solvent, and, especially, air, suitably using a catalyst, for example palladium or platinum. The reaction suitably takes place in an inert solvent in the presence of an organic acid, for example acetic acid; the organic acid can serve directly as the solvent. The reaction temperature is in general 20-200 °C, preferably 30-100 °C. In a particularly preferred method for oxidising the 1,4-dihydropyridine-2,3 carboxylic acid derivatives, a reaction mixture containing an equimolar quantity of manganese dioxide in acetic acid is maintained at a temperature of 50-100 °C for ½ to 1 hour.

Examples 1a and 2 of EP-A-172140 describe the preparation of a series of compounds of formula III by deamination of tetrahydropyridines of formula II in the presence of silica gel and toluene under reflux. The resulting dihydropyridine compounds of formula III are isolated. Examples 1b and 3 then describe the oxidation of these compounds to the pyridines of formula I, using air/acetic acid at 45 °C (Example 1b), and manganese dioxide/acetic acid at 60 °C (Example 3).

In accordance with the present invention there is provided a process for the preparation of a pyridine-2,3-dicarboxylic acid or a derivative thereof of the formula I

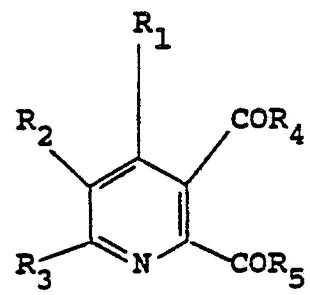

(I)

in which $R_1$ represents a hydrogen atom, or an optionally substituted alkyl, alkylthio, alkoxy, phenyl or phenoxy group, $R_2$ has the same meaning as $R_1$ and additionally represents a chlorine, fluorine or bromine atom, $R_3$ represents a hydrogen atom or an alkyl group, or together with $R_2$ represents a trimethylene or tetramethylene group, each of $R_4$ and $R_5$ independently represents a group of formula -OH, $-NH_2$, $-NHR_6$, $-NR_6R_7$ or $-OR_8$, wherein each of $R_6$ and $R_7$ independently represents an alkyl, cycloalkyl, allyl, methallyl or propargyl group, or an optionally substituted aryl or aralkyl group, or $R_6$ and $R_7$ together represent an alkylene or oxaalkylene chain, and $R_8$ represents an alkyl, cycloalkyl, allyl, methallyl or propargyl group or an optionally substituted aralkyl or aryl group, or $R_4$ and $R_5$ together represent a moiety of formula -O- or $-NR_{10}-$, wherein $R_{10}$ represents a hydrogen atom, or an optionally substituted alkyl, allyl, methallyl, propargyl, cycloalkyl, aryl or aralkyl group, comprising deaminating a 1-amino-1,2,3,4-tetrahydro-pyridine-2,3- carboxylic acid, or derivative thereof, of the general formula II

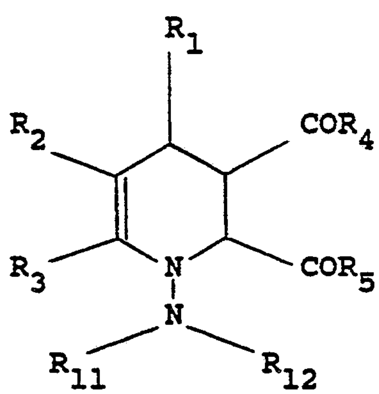

(II)

wherein $R_1$, $R_2$, $R_3$, $R_4$ and $R_5$ are as defined above and wherein each of $R_{11}$ and $R_{12}$ independently represents an alkyl, cycloalkyl, aralkyl or aryl group, or together represent an alkylene or oxaalkylene chain, wherein the compound of general formula I is prepared from the compound of general formula II in a single

step or procedure under oxidising conditions.

The 'one-pot' process of the present invention thus eliminates the need, in accordance with the prior art, of carrying out a first step or procedure to prepare the dihydropyridine compound, isolating that compound, and then carrying out a second step or procedure to prepare from it the pyridine.

The process of the invention suitably employs the following conditions:

- an oxidising agent selected from a gas comprising free oxygen, for example oxygen itself or air; a peroxide oxidising agent, for example hydrogen peroxide, a manganese-containing oxidising agent, for example manganese dioxide, a chromium-containing oxidising agent, for example chromic acid (suitably as sodium or potassium chromate in an organic acid), iodine in an organic solvent, a cerium-containing oxidising agent, for example ceric ammonium nitrate, or a selenium-containing oxidising agent, for example selenium dioxide. Manganese dioxide is a preferred oxidising agent. Gaseous oxidation is suitably effected in the presence of a catalyst, for example palladium or platinum. The procedure is preferably effected in the presence of an organic acid, suitably acetic acid, and optionally, an organic solvent and/or water. Suitable organic solvents include polar organic solvents, for example organic acids, ethers, halogenated hydrocarbons, esters and lactones, amides and lactams, sulphones and sulphoxides, tertiary amines, substituted benzene compounds, nitriles and alcohols (such solvents are defined in more detail in EP-A-172,140 page 5 line 13 to page 6 line 7). An organic acid, for example acetic acid, preferably glacial, can serve as both the solvent and the acid. The reaction temperature may be in the range 20-200°C, preferably 30-100°C, and most preferably 50-100°C. The reaction time is normally $\frac{1}{2}$ to 4 hours.

The reaction conditions for the single step of the present invention are thus similar to those of EP-A-172140 for the second of the two steps required in accordance with the process of EP-A-172140 to prepare the pyridine compounds I from tetrahydropyridine compounds II.

In general terms, and unless otherwise stated in this specification an alkyl moiety may be linear or branched and preferably has 1-6, especially 1-4 carbon atoms; an alkylene or oxaalkylene moiety may be linear or branched and preferably has 2-6 carbon atoms; a cycloalkyl group preferably has 3-10 carbon atoms, especially 3-6 carbon atoms; an aryl group is preferably phenyl; and an aralkyl group is preferably phenyl-$C_{1-4}$alkyl, especially benzyl.

More specifically, $R_1$ and $R_2$ are suitably independently selected from hydrogen; alkyl, alkylthio or alkoxy, any of which may be optionally substituted by one or more moieties selected from halogen atoms and hydroxy, $C_{1-4}$alkoxy, phenyl, phenoxy, cyano, carboxyl, and ($C_{1-4}$alkoxy)carbonyl; or phenyl or phenoxy either of which may be optionally substituted by one or moieties independently selected from halogen, cyano, $C_{1-4}$ cyanoalkyl. Preferably, $R_1$ represents a hydrogen atom and $R_2$ represents a hydroxyalkyl, haloalkyl or, especially, an alkyl group, most preferably methyl or ethyl.

$R_3$ preferably represents a hydrogen atom.

In relation to the groups $R_4$ and $R_5$, the groups $R_6$, $R_7$ and $R_8$ which may be contained therein each preferably represents alkyl, cyclopentyl or cyclohexyl, or phenyl or benzyl either of which may be optionally ring-substituted by one or more moieties selected from $C_{1-4}$alkyl, $C_{1-4}$alkoxy or halogen; or $R_6$ and $R_7$ may together represent pentamethylene, tetramethylene or 3-oxapentylene. The group $R_{10}$ preferably represents a hydrogen atom; or an alkyl group optionally substituted by one or more moieties independently selected from hydroxy, $C_{1-4}$alkoxy, phenoxy, carboxy, ($C_{1-4}$alkoxy)carbonyl, phenoxycarbonyl, benzyloxycarbonyl, aminocarbonyl, mono- or di-($C_{1-4}$alkyl)aminocarbonyl, halogen and cyano; or represents a cyclopentyl or cyclohexyl group; or a phenyl or benzyl group optionally ring-substituted by one or more moieties selected from $C_{1-4}$alkyl, $C_{1-4}$alkoxy or halogen. $R_{10}$ is especially preferred as an alkyl, cyanoalkyl, aminocarbonylalkyl or phenyl group. A particularly preferred series of groups $R_{10}$ may be defined by the formula

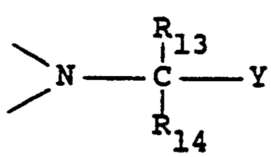

in which Y represents -CN or -$CONH_2$ and each $R_{13}$ and $R_{14}$ independently represents hydrogen or, preferably, alkyl.

$R_4$ and $R_5$ preferably represent, as separate moieties, -OH or -$OR_8$, especially -OH or -O-$C_{1-4}$ alkyl, conveniently being identical, or as a joint moiety, -O- or -$NR_{10}$-, especially -O-,

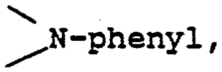, 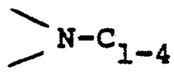, 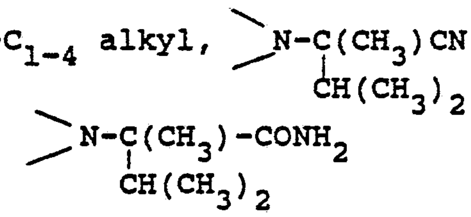

or

R₁₁ and R₁₂ each preferably represents an alkyl, cyclopentyl, cyclohexyl, phenyl or benzyl group, or, jointly, a tetramethylene, pentamethylene or 3-oxapentylene group. Preferably, $R_{11}$ and $R_{12}$ each represents a methyl, ethyl, phenyl or benzyl group, most preferably a methyl group.

The preparation of the starting compounds of formula II is suitably carried out by reacting compounds of formula IV and V

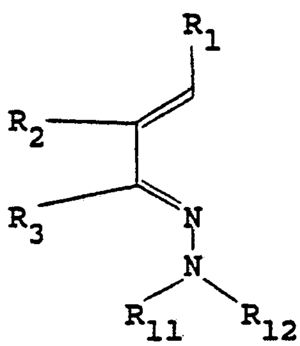 (IV) 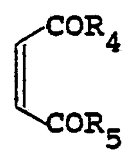 (V)

where $R_1$, $R_2$, $R_3$, $R_4$, $R_5$, $R_{11}$ and $R_{12}$ are as defined above, at elevated temperature, suitably 80-120° C, under an inert atmosphere, preferably nitrogen. Such a reaction is described in Tetrahedron Letters, 1982, 23 (32), p. 3261-4. However, it has now, surprisingly, been determined that reaction is complete after 24 hours and yields the tetrahydropyridine of formula II as a cis/trans mixture with regard to the separate $COR_4$/$COR_5$ substituents.

Compounds of the general formula I are useful chemical intermediates, in particular for the preparation of the herbicidal pyridyl imidazolinone compounds of the type described in EP-A-41623 and EP-A-41624.

The invention will now be further described with reference to the following Examples.

Example 1

Preparation of dimethyl 5-methylpyridine-2,3-di-carboxylate

a) Dimethyl maleate (67.5g) and 2-methylprop-2-enal dimethyl hydrazone (105.1g) were mixed and heated at 100° C for 24 hours, and allowed to cool to ambient temperature. Excess hydrazone was removed by evaporation under vacuum as the temperature was raised to 70° C. Analysis by NMR showed that the crude product, approximately 120g, contained a mixture of cis/trans (3:4) dimethyl 1-dimethylamino-5-methyl-1,2,3,4,-tetrahydropyridine-2,3-dicarboxylate.

Analysis
Calculated 56.2%C 7.8%H 10.9%N
Found 56.4%C 7.8%H 11.0%N

b) to the crude product of a) above in glacial acetic acid (750g) at 50° C was added manganese dioxide (40.5g), portionwise over 40 minutes. The temperature was increased to 70° C and maintained at 70-80° C for 30 minutes. The reaction mixture was then allowed to cool slightly, being maintained at 60-70° C for about 1 hour, with stirring. The solvent was removed by evaporation and the residue passed through a large florisil column, using dichloromethane as eluant. The crude product was split into 2 portions and purified by flash chromatography, using silica /ethyl acetate-hexane (40:60). The title compound was

produced as an orange oil which crystallised on cooling.
Yield 48.7g (50% based on maleate).

Analysis

Calculated 57.4%C 5.3%H 6.7%N

Found 57.3%C 6.0%H 6.6%N

The identity of the product was further confirmed by NMR and mass spectral analysis

## Example 2

### Preparation of dimethyl 5-ethylpyridine-2,3-dicarboxylate

(a) 2-Ethylprop-2-enal dimethyl hydrazone (91g) and dimethyl maleate (52g) were mixed and heated at 100°C under a nitrogen atmosphere. Reaction was complete after 4 days. Excess hydrazone was removed by evaporation yielding a crude mixture of the cis/trans isomers (2:3) of dimethyl 1-dimethylamino-5-ethyl-1,2,3,4-tetrahydropyridine-2,3-dicarboxylate (98g, an orange oil).

Analysis

Calculated 57.8%C 8.1%H 10.4%N

Found 57.8%C 8.6%H 10.4%N

(b) The crude product of Example 2(a) was dissolved in acetic acid (900ml) and warmed to 70°C. Manganese dioxide (30.8g) was added in portions over 20 minutes. The reaction mixture was maintained at 70-80°C for 30 minutes. The reaction mixture was cooled and the solvent was evaporated off. The residue was carefully added to aqueous sodium bicarbonate, extracted with chloroform, washed with sodium bicarbonate and water, and dried ($Na_2SO_4$). The residue was evaporated to leave a crude red oil, which was distilled (Kugelrohr) using a pot temperature of 110-120°C and a pressure of approximately 0.04 mbar. The title compound was isolated as a pale yellow liquid (yield 47.7g, 60% yield based on maleate).

Analysis

Calculated 59.2%C 5.8%H 6.3%N

Found 59.3%C 6.0%H 6.6%N

The identity of the product was further confirmed by NMR analysis.

## Example 3

### Preparation of

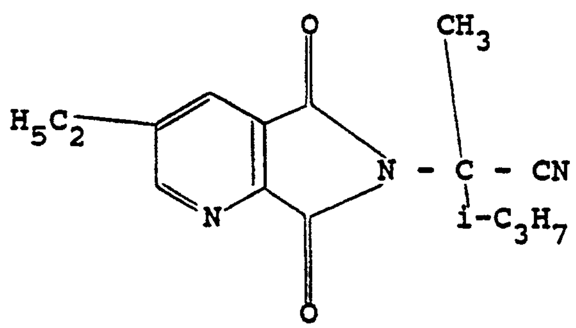

The compound of formula II wherein $R_1 = H$, $R_2 = C_2H_5$, $R_3$-H, $R_{11} = CH_3$, $R_{12} = CH_3$ and $-(R_4 + R_5)- = N-C(CH_3)\ (i-C_3H_7)\ (CN)$ (6g) was dissolved in acetic acid (48ml). The mixture was warmed to 70°C and manganese dioxide (2.0g) added in portions over 30 minutes. The temperature was maintained at 70-80°C for 3½ hours. The reaction mixture was filtered, the solvent evaporated, and the residue added to 10% aqueous sodium bicarbonate. The product was extracted into chloroform (2×60ml), the organic layer dried ($Na_2SO_4$), and evaporated to leave an oily, red residue. Purification by flash chromatography (on silica gel using 1:1 (v/v) ethyl acetate/hexane as eluant) yielded the title compound as a red oil which solidified (3.8g, 74% based on the compound of formula II).

Analysis

Calculated 66.4%C 6.3%H 15.5%N

Found 66.5%C 6.5%H 15.2%N

The structure of the compound was further confirmed by NMR and mass spectrometry.

## Claims

1. A process for the preparation of a pyridine-2,3-dicarboxylic acid or derivative thereof, of the formula I

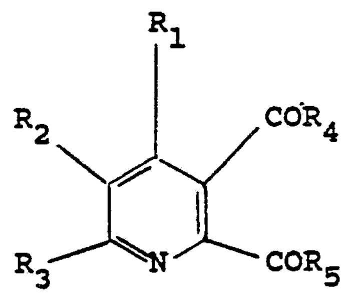

(I)

in which $R_1$ represents a hydrogen atom, or an optionally substituted alkyl, alkylthio, alkoxy, phenyl or phenoxy group, $R_2$ has the same meaning as $R_1$ and additionally represents a chlorine, fluorine or bromine atom, $R_3$ represents a hydrogen atom or an alkyl group or together with $R_2$ represents a trimethylene or tetramethylene group, each of $R_4$ and $R_5$ independently represents a group of formula -OH, $-NH_2$, $-NHR_6$, $-NR_6R_7$, or $-OR_8$, wherein each of $R_6$ and $R_7$ independently represents an alkyl, cycloalkyl, allyl, methallyl or propargyl group, or an optionally substituted aryl or aralkyl group, or $R_6$ and $R_7$ together represent an alkylene or oxaalkylene chain, and $R_8$ represents an alkyl, cycloalkyl, allyl, methallyl or propargyl group or an optionally substituted aralkyl or aryl group, or $R_4$ and $R_5$ together represent a moiety of formula -O- or $-NR_{10}-$, wherein $R_{10}$ represents a hydrogen atom, or an optionally substituted alkyl, allyl, methallyl, propargyl, cycloalkyl, aryl or aralkyl group, comprising deaminating a 1-amino-1,2,3,4-tetrahydropyridine-2,3- carboxylic acid, or derivative thereof, of the general formula II

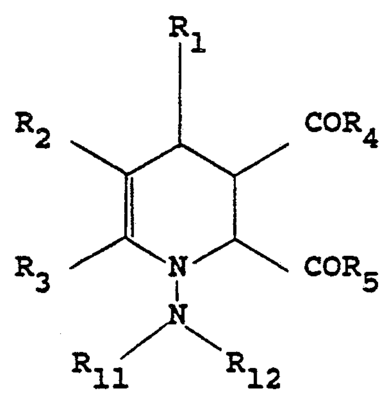

(II)

wherein $R_1$, $R_2$, $R_3$, $R_4$ and $R_5$ are as defined above and wherein each of $R_{11}$ and $R_{12}$ independently represents an alkyl, cycloalkyl, aralkyl, aralkyl or aryl group, or together represent an alkylene or oxaalkylene chain, characterised in that the compound of general formula I is prepared from the compound of general formula II in a single step or procedure under oxidising conditions.

2. A process according to claim 1, wherein the single step or procedure takes place at a temperature in the range 20-200°C, in the presence of an organic acid and using air, oxygen, hydrogen peroxide, manganese dioxide, chromic acid, ceric ammonium nitrate or selenium dioxide as oxidising agent.

3. A process according to claim 2, wherein the single step or procedure takes place at a temperature in the range 30-100°C, in the presence of acetic acid and manganese dioxide.

4. A process according to any one of the preceding claims wherein $R_1$ and $R_2$ are independently selected from hydrogen atoms; alkyl, alkylthio or alkoxy groups, optionally substituted by one or more moieties selected from halogen atoms and hydroxy, $C_{1-4}$alkoxy, phenyl, phenoxy, cyano, carboxy, and ($C_{1-4}$alkoxy)carbonyl groups; or phenyl or phenoxy groups optionally substituted by one or more moieties independently selected from halogen, cyano, $C_{1-4}$alkyl, $C_{1-4}$alkoxy, $C_{1-4}$haloalkyl and $C_{1-4}$cyanoalkyl groups.

5. A process according to any one of the preceding claims wherein $R_3$ represents a hydrogen atom.

6. A process according to any one of the preceding claims, wherein each of $R_4$ and $R_5$ independently represents a group of formula -OH, $-NH_2$, $-NHR_6$, $-NR_6R_7$, or $-OR_8$, where each of $R_6$, $R_7$ and $R_8$ independently represents an alkyl, cyclopentyl or cyclohexyl group, or a phenyl or benzyl group optionally

ring-substituted by one or more moieties selected from $C_{1-4}$alkyl or $C_{1-4}$alkoxy groups and halogen atoms; or $R_6$ and $R_7$ may together represent pentamethylene, tetramethylene or 3-oxapentylene; or $R_4$ and $R_5$ together represent a moiety of formula -O- or -$NR_{10}$- where $R_{10}$ represents a hydrogen atom or an alkyl group optionally substituted by one or more moieties independently selected from hydroxy, $C_{1-4}$alkoxy, phenoxy, carboxy, ($C_{1-4}$alkoxy)carbonyl, phenoxycarbonyl, benzyloxycarbonyl, aminocarbonyl, mono- or di-($C_{1-4}$alkyl)aminocarbonyl, halogen and cyano; or represents a cyclopentyl or cyclohexyl group; or a phenyl or benzyl group optionally ring-substituted by one or more moieties selected from $C_{1-4}$alkyl and $C_{1-4}$alkoxy groups and halogen atoms.

7. A process according to claim 6, wherein each of $R_4$ and $R_5$ independently represents a group of the formula -OH or -$OR_8$, where $R_8$ represents an alkyl group, or jointly represent -O- or -$NR_{10}$ where $R_{10}$ represents an alkyl, cyanoalkyl, aminocarbonylalkyl or phenyl group.

8. A process according to any one of the preceding claims, wherein each of $R_{11}$ and $R_{12}$ independently represents an alkyl, cyclopentyl, cyclohexyl, phenyl or benzyl group, or, jointly, a tetramethylene, pentamethylene or 3-oxapentylene group.

9. A process according to any one of claims 1 to 8, in which the compound of formula II has been prepared by reacting compounds of formulae IV and V

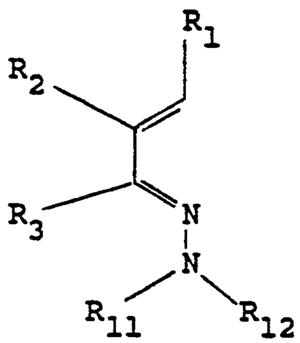
(IV)

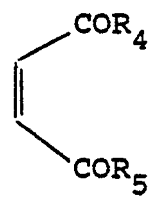
(V)

where $R_1$, $R_2$, $R_3$, $R_4$, $R_5$, $R_{11}$ and $R_{12}$ are as defined in any one of claims 1, 4, 5, 6, 7, or 8 at an elevated temperature for a period not in excess of 30 hours, to produce a compound of formula II.

10. A compound of the general formula I, whenever prepared by a process as claimed in any one of the preceding claims.

European Patent Office

# EUROPEAN SEARCH REPORT

Application Number

EP 88 20 0717

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D<br>A | EP-A-0 172 140 (CIBA-GEIGY AG)<br>* example 3, table, pages 17-18 *<br>* whole document * | 10<br>1-9 | C 07 D 213/79<br>C 07 D 213/80<br>C 07 D 213/81<br>C 07 D 215/48<br>C 07 D 221/04<br>C 07 D 471/04<br>C 07 D 491/04<br>C 07 D 211/98 //<br>(C 07 D 471/04<br>C 07 D 221:00<br>C 07 D 209:00 )<br>(C 07 D 491/04<br>C 07 D 307:00<br>C 07 D 209:00 ) |
| X<br>A | EP-A-0 161 221 (CIBA-GEIGY AG)<br>* examples 2-6; tables pages 13-16, 18, 21 *<br>* claim 1 * | 10<br>1 | |
| A | JOURNAL OF THE CHEMICAL SOCIETY, CHEMICAL COMMUNICATIONS, no. 2, 1984, pages 114-116; K.T. POTTS et al.: "Regiochemical control in Diels-Alder routes to aza-arthraquinone derivatives"<br>* page 115, scheme and column 1, lines 15-23 * | 1 | |
| | | | **TECHNICAL FIELDS SEARCHED (Int. Cl.4)** |
| | | | C 07 D 213/00<br>C 07 D 215/00<br>C 07 D 221/00<br>C 07 D 471/00<br>C 07 D 491/00<br>C 07 D 211/00 |

The present search report has been drawn up for all claims

| Place of search | Date of completion of the search | Examiner |
|---|---|---|
| BERLIN | 24-08-1988 | VAN AMSTERDAM L.J.P. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document